# FASCICULE DE BREVET EUROPEEN

(11) **EP 1 089 716 B1**
(45) Date de publication et mention de la délivrance du brevet: **24.07.2002**
(21) Numéro de dépôt: 99957145.8
(22) Date de dépôt: 22.06.1999
(51) Int. Cl.: A61K 9/20, A61K 9/48

(54) **ELEMENTS INGERABLES**
EINNEHMBARE PRODUKTE
INGESTIBLE ELEMENTS

(30) Priorité: 22.06.1998 FR 9807838
(43) Date de publication de la demande: 11.04.2001
(73) Titulaire: Douaire, Philippe, 750004 Paris (FR); Vincensini, Sandrine, 75012 Paris (FR); Collard, Jean Claude, 78400 Chatou (FR)
(72) Inventeur: Douaire, Philippe, 75004 Paris (FR); Vincensini, Sandrine, 75012 Paris (FR)
(74) Mandataire: Doireau, Marc
(86) Numéro de dépôt international: FR9901489
(87) Numéro de publication internationale: WO9966905

(56) Documents cités:
- EP-A- 0 214 834
- EP-A- 0 259 219
- DE-C- 4 342 146
- FR-A- 2 137 872
- MARIA EDVIGE SANGALLI ET AL: "INERT MONOLITHIC DEVICE WITH A CENTRAL HOLE FOR CONSTANT DRUG RELEASE" EUROPEAN JOURNAL OF PHARMACEUTICS AND BIOPHARMACEUTICS, vol. 40, no. 6, 1 décembre 1994 (1994-12-01), pages 370-373, XP000480157
- CLEAVE J.P.: "Some geometrical considerations concerning the design of tablets" J. PHARM. PHARMACOL., vol. 17, 1965, pages 698-702, XP002096313
- LIST P.H. ET AL: "Hagers Handbuch der Pharmazeutischen Praxis" 1971 , SPRINGER VERLAG , BERLIN HEIDELBERG NEW YORK XP002096314 page 691; figure 388A; exemple Q

## Description

La présente invention se rapporte principalement aux éléments susceptibles d'être ingérés tels que bonbons, fruits secs, cacahuètes ou médicaments à prise par voie orale.

Des médicaments comme par exemple des comprimés, des pilules ou des gélules, ainsi que certains denrées alimentaires comme par exemple des cacahuètes, des noisettes, des bonbons, des glaçons, des morceaux de sucre, des confiseries et des chocolats se présentent sous la forme d'une pièce solide relativement dure qui, avalée de travers, notamment par une personne âgée ou un enfant, et plus particulièrement un jeune enfant, risque d'obturer les voies respiratoires provoquant un étouffement.

Il est connu du document FR-A-2 137 872 la réalisation d'un canal dans les pâtes alimentaires pour fabriquer des nouilles, sans indication d'utilisation particulière.

Il est connu de l'article paru dans European journal of pharmaceutics and biopharmaceutics, vol. 40, n° 6, pages 370 - 373, ou des documents EP-A-0 214 834, EP A-0 259 219, et DE 43 42 146 C, de réaliser un canal central dans des comprimés pour, respectivement, améliorer la dissolution du produit actif, rendre sa diffusion sensiblement constante, contrôler sa libération, ou permettre le partage du comprimé en plusieurs sections.

Aucun de ces documents ne concerne les problèmes liés à l'étouffement.

C'est par conséquent un but de la présente invention d'offrir des éléments ingérables ne présentant pas de risque d'étouffement en cas de fausse route alimentaire, c'est-à-dire lorsqu'ils sont avalés de travers.

C'est également un but de la présente invention d'offrir de telles pièces ingérables ayant une forme les rendant plus faciles à avaler.

C'est aussi un but de la présente invention d'offrir des médicaments présentant des propriétés de libération des principes actifs optimisées en fonction du traitement prescrit.

Ces buts sont atteints selon la présente invention par un élément, du type médicament, bonbon, cacahuète, produit de charcuterie sèche, morceau de fromage ou analogue dans lequel est ménagé au moins un canal de circulation d'air.

L'invention sera mieux comprise au moyen de la description ci-après et des figures annexées données comme des exemples non limitatifs et sur lesquels :
- la figure 1 est une vue en perspective d'un premier exemple de réalisation d'un comprimé selon la présente invention ;
- la figure 2 est une vue en perspective d'un deuxième exemple de réalisation d'un comprimé selon la présente invention ;
- la figure 3 est une vue en perspective de l'exemple préféré de réalisation d'un comprimé selon la présente invention ;
- la figure 4 est une vue en perspective de l'exemple préféré de réalisation d'une gélule selon la présente invention avant assemblage ;
- la figure 5 est une vue analogue de la gélule de la figure 4 après assemblage ;
- la figure. 6 est une vue en coupe d'un comprimé enrobé selon la présente invention ;
- la figure 7 est une vue de côté d'une chaîne de réalisation de canaux de circulation d'air dans des éléments ingérables selon la présente invention.

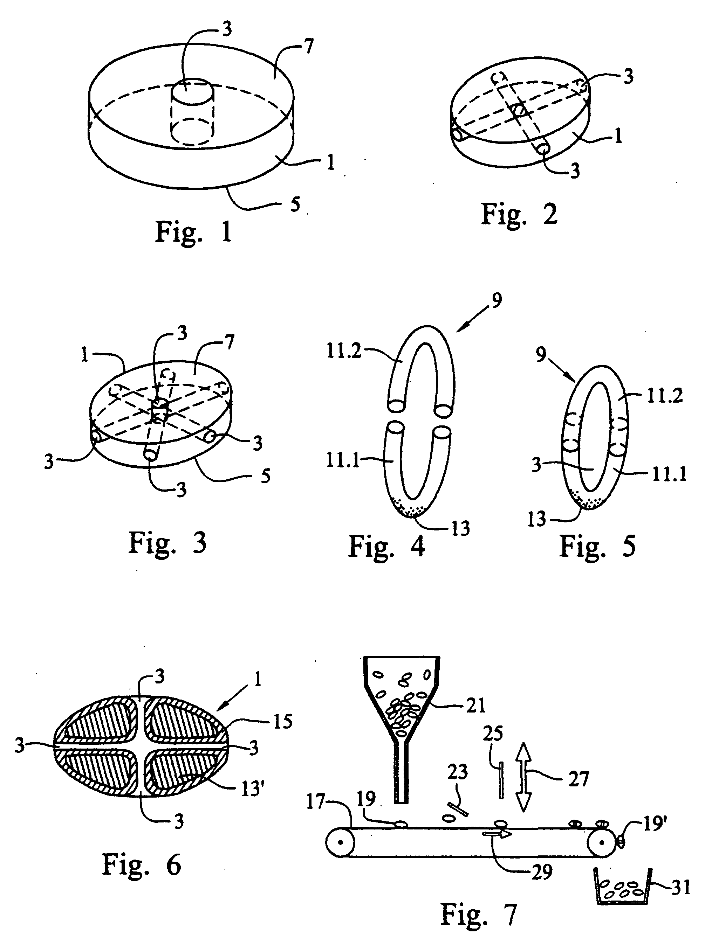

Sur les figures 1 à 7, on a utilisé les mêmes références pour désigner les mêmes éléments.

Sur la figure 1, on peut voir un comprimé 1 d'un principe actif comme par exemple de l'aspirine, dans l'épaisseur duquel a été ménagé un canal 3 reliant deux faces principales 5 et 7 du comprimé. Le canal 3 permet la circulation de l'air. Ainsi, si le comprimé 1 venait à être immobilisé et à obturer la trachée artère, le canal 3 permettrait le passage de l'air et éviterait l'étouffement dans l'attente de l'évacuation du cachet des voies respiratoires. Le canal 3 est par exemple cylindrique et a par exemple un diamètre compris entre 0,5 mm et 6 mm, de préférence compris entre 2 mm et 4 mm, par exemple égal à 2 mm, 3 mm ou 4 mm. Toutefois, il est bien entendu que la mise en oeuvre de canaux ayant des sections polygonales régulières ou non ou autres, ainsi que des canaux ayant des sections variables ne sort pas du cadre de la présente invention.

Sur la figure 2, on peut voir un comprimé 1 selon la présente invention comportant une pluralité de canaux 3 répartis sur le pourtour avantageusement connectés, par exemple au centre du comprimé. Le comprimé de la figure 3 a une forme plus ramassée le rendant plus facile à avaler et de plus est muni des canaux 3 régulièrement répartis sur son pourtour complété par un canal 3 reliant ses faces principales 5 et 7. Tous les canaux sont interconnectés au centre du comprimé 1. Ainsi, quelque soit la position d'immobilisation du comprimé dans la trachée artère, les poumons sont toujours reliés par un canal de circulation d'air au milieu extérieur contenant de l'air.

Sur les figures 4 et 5, on peut voir une gélule 9 selon la présente invention dont la capsule est composée d'un premier tube en U 11.1 ayant un diamètre externe sensiblement égal au diamètre interne d'un second tube en U 11.2. Les tubes 11.1 et 11.2 sont susceptibles d'être emboîtés l'un dans l'autre, de manière à former un tube fermé sur lui-même, susceptible de recevoir des particules micro-encapsulées des principes actifs du médicament. L'espace entre les parois du tube forment un canal 3 de passage de l'air. La gélule 9 peut être sensiblement torique ou au contraire aplatie. En variante, chacun des tubes 11.1 et 11.2 a un diamètre variable, de manière à ce que le diamètre externe de l'extrémité resserrée d'un premier tube corresponde au diamètre interne de l'extrémité évasée de l'autre, permettant leur emboîtement mutuel.

En variante, la capsule de la gélule est constituée par un tube unique dont les extrémités sont mutuellement emboîtées.

Sur la figure 6, on peut voir un exemple de comprimé 1 pelliculé ou enrobé selon la présente invention comportant un principe actif 13' recouvert par une pellicule 15 destinée à en éviter le contact avec les papilles gustatives (dans le cas où le médicament a un goût désagréable) ou pour contrôler la libération du principe actif. La pellicule 15 est par exemple à base de sucre. Avantageusement, la pellicule 15 recouvre complètement le principe actif 13' y compris au niveau des canaux 3.

Il est bien entendu que la présente invention n'est pas limitée aux médicaments mais s'applique également aux denrées alimentaires comme par exemple aux bonbons, gâteaux secs, noix, cacahuètes, produits de charcuterie sèche, morceaux de fromages, glaçons, morceaux de sucre, confiseries, chocolats, produits extrudés du type gâteaux apéritifs et analogues. Les éléments peuvent être obtenus par compression ou par moulage dans des moules comportant des réserves correspondant aux canaux 3. Ces réserves peuvent être par exemple réalisées sous forme de tiges fixes ou mobiles lorsque cela est nécessaire pour permettre le démoulage.

Pour les produits tels que noisettes, cacahuètes, noix de cajou ou analogues ne subissant pas de mise en forme, les canaux 3 sont avantageusement réalisés par ablation de matière mécanique ou thermique. L'ablation de matière est avantageusement effectuée à l'emporte-pièce bien qu'une ablation par percement, usinage mécanique ou par rayons laser ne sorte pas du cadre de la présente invention.

Sur la figure 7, on peut voir une chaîne de réalisation de canaux 3 par ablation de matière avantageusement à l'emporte-pièce. La chaîne comporte un convoyeur 17 alimenté en éléments ingérables 19 par un dispositif d'alimentation 21, par exemple une trémie. Un dispositif de guidage mécanique symbolisé en 23 assure l'orientation des éléments 19, notamment des noisettes, des cacahuètes ou, avantageusement, des moitiés de cacahuètes.

Les éléments dans lesquels on veut ménager des canaux 3 arrivent sous un emporte-pièce 25 (ou un autre outil d'ablation) entraîné dans un mouvement symbolisé par la flèche 27, perpendiculaire au sens du déplacement 29 du convoyeur 17 et synchronisé avec ce dernier. Dans un premier exemple de réalisation, l'emporte-pièce 25 travaille à la volée, le convoyeur 17 ayant un mouvement continu. En variante, le convoyeur assure l'amenée d'un élément 19 sous l'emporte-pièce 25 puis s'immobilise pendant la manoeuvre de celui-ci. Le mouvement du convoyeur 17 reprend et les éléments 19 munis de leurs canaux 3 sont recueillis dans un réceptacle 31 en vue de leur conditionnement.

La présente invention s'applique à l'industrie agro-alimentaire ainsi qu'à l'industrie pharmaceutique.

## Revendications

1. **Utilisation** d'au moins un canal dans un élément susceptible d'être ingéré, **caractérisée en ce que** le canal (3) sert de passage de l'air pour éviter l'étouffement en cas de fausse route alimentaire.

2. **Utilisation** selon la revendication 1, **caractérisée en ce que** le canal (3) est cylindrique ou sensiblement cylindrique.

3. **Utilisation** selon la revendication 2, **caractérisée en ce que** le canal (3) a un diamètre compris entre 0,5 mm et 6 mm, de préférence entre 1 mm et 4 mm, de manière préférée entre 2 mm et 3 mm.

4. **Utilisation** selon l'une quelconque des revendications précédentes, **caractérisée en ce que** ledit élément est un médicament à prise par voie orale.

5. Utilisation selon la revendication 4, **caractérisée en ce que** ledit élément est un comprimé.

6. **Utilisation** selon la revendication 4 ou 5, **caractérisée en ce que** ledit élément est un comprimé enrobé.

7. **Utilisation** selon la revendication 6, **caractérisé en ce que** l'enrobage recouvre les parois du canal (3) de passage d'air.

8. **Utilisation** selon la revendication 4, **caractérisée en ce que** ledit élément est une gélule.

9. **Utilisation** selon la revendication 8, **caractérisée en ce que** la gélule comporte une capsule comportant un élément tubulaire fermé sur lui-même.

10. **Utilisation** selon l'une quelconque des revendications 1 à 3, **caractérisée en ce que** ledit élément est un aliment.

11. **Utilisation** selon la revendication 10, **caractérisée en ce que** l'aliment est une pièce solide prise parmi les cacahuètes, les noisettes, les bonbons, les glaçons, les morceaux de sucre, les confiseries et les chocolats.

## Patentansprüche

1. Verwendung mindestens eines Kanals in einem zum Einnehmen vorgesehenen Element, **dadurch gekennzeichnet, dass** der Kanal (3) zum Durchtritt von Luft dient, um eine Erstickung im Falle des Verschluckens zu vermeiden.

2. Verwendung nach Anspruch 1, **dadurch gekennzeichnet, dass** der Kanal (3) zylindrisch oder im Wesentlichen zylindrisch ist.

3. Verwendung nach Anspruch 2, **dadurch gekennzeichnet, dass** der Kanal einen Durchmesser von 0,5 bis 6 mm, vorzugsweise 1 bis 4 mm, vorzugsweise 2 bis 3 mm aufweist..

4. Verwendung nach einem der vorhergehenden Ansprüche, **dadurch gekennzeichnet, dass** das Element ein oral zu verabreichendes Medikament ist.

5. Verwendung nach Anspruch 4, **dadurch gekennzeichnet, dass** das Element eine Tablette ist.

6. Verwendung nach Anspruch 4 oder 5, **dadurch gekennzeichnet, dass** das Element eine überzogene Tablette ist.

7. Verwendung nach Anspruch 6, **dadurch gekennzeichnet, dass** der Überzug die Wände des Kanals (3) für den Luftdurchtritt bedeckt.

8. Verwendung nach Anspruch 4, **dadurch gekennzeichnet, dass** das Element eine Gelatinekapsel ist.

9. Verwendung nach Anspruch 8, **dadurch gekennzeichnet, dass** die Gelatinekapsel eine Kapsel umfasst, welche ein in sich schlauchförmiges Element umfasst.

10. Verwendung nach einem der Ansprüche 1 bis 3, **dadurch gekennzeichnet, dass** das Element ein Nahrungsmittel ist.

11. Verwendung nach Anspruch 10, **dadurch gekennzeichnet, dass** das Nahrungsmittel ein Festkörper, ausgewählt aus Erdnüssen, Nüssen, Bonbons, Eisbonbons, Zuckerstücken, Süßwaren und Konfekt, ist.

## Claims

1. Use of at least one channel in an element suitable for being ingested, the use being **characterized in that** the channel (3) acts as an air passage for avoiding choking in the event of the element being swallowed the wrong way.

2. The use according to claim 1, **characterized in that** the channel (3) is cylindrical or substantially cylindrical.

3. The use according to claim 2, **characterized in that** the channel (3) has a diameter lying in the range 0.5 mm to 6 mm, preferably in the range 1 mm to 4 mm, and more preferably in the range 2 mm to 3 mm.

4. The use according to any preceding claim, **characterized in that** said element is a medicine for taking orally.

5. The use according to claim 4, **characterized in that** said element is a tablet.

6. The use according to claim 4 or 5, **characterized in that** said element is a coated tablet.

7. The use according to claim 6, **characterized in that** the coating covers the walls of the air-passing channel (3).

8. The use according to claim 4, **characterized in that** said element is a capsule.

9. The use according to claim 8, **characterized in that** the capsule comprises an outer skin in the form of a tubular element that is closed to form a loop.

10. The use according to any one of claims 1 to 3, **characterized in that** said element is a foodstuff.

11. The use according to claim 10, **characterized in that** the foodstuff is a solid piece selected from: nuts; sweets; candies; ice cubes; sugar lumps; confectionery; and chocolates.
